# EUROPÄISCHE PATENTSCHRIFT

(11) **EP 2 379 142 B1**
(45) Veröffentlichungstag und Bekanntmachung des Hinweises auf die Patenterteilung: **09.09.2015**
(21) Anmeldenummer: 09799074.1
(22) Anmeldetag: 10.12.2009
(51) Int. Cl.: A61M 15/00

(54) **DOSIERVORRICHTUNG ZUR ERZEUGUNG EINES GASSTROMES MIT EINEM IN DIESEM FEIN VERTEILTEN WIRKSTOFF**
DOSING DEVICE FOR GENERATING A GAS FLOW WITH AN ACTIVE SUBSTANCE FINELY DISPERSED IN THE LATTER
DISPOSITIF DE DOSAGE POUR LA PRODUCTION D'UN COURANT GAZEUX PRÉSENTANT UNE SUBSTANCE ACTIVE FINEMENT RÉPARTIE DANS LEDIT COURANT GAZEUX

(30) Priorität: 23.12.2008 EP 08172765
(43) Veröffentlichungstag der Anmeldung: 26.10.2011
(73) Patentinhaber: Sanofi SA, 1214 Vernier (CH)
(72) Erfinder: MAYER, Stefan, 79098 Freiburg im Breisgau (DE); KAMLAG, Yorick, 82396 Paehl (DE)
(74) Vertreter: Epping - Hermann - Fischer
(86) Internationale Anmeldenummer: PCT/EP2009/066829
(87) Internationale Veröffentlichungsnummer: WO 2010/072575

(56) Entgegenhaltungen:
- EP-A- 1 905 472
- EP-A- 1 905 473

## Beschreibung

Die Erfindung liegt auf dem Gebiet der Medizintechnik, ist jedoch auch anderweitig vorteilhaft einsetzbar.

Die Erfindung betrifft speziell eine Dosiervorrichtung zur Erzeugung eines Gasstromes mit einem in diesem fein verteilten Wirkstoff.

Derartige Dosiervorrichtungen werden insbesondere zur Inhalation von Wirkstoffen eingesetzt. Mit besonderem Vorteil lassen sich solche Vorrichtungen zum Inhalieren pulverförmiger Substanzen einsetzen, die in einer Vorratskammer angeordnet und mittels einer Dosiereinrichtung für einmaliges Inhalieren entnommen und in einen Saugluftstrom eingebracht werden können. Dies kann beispielsweise durch Abnehmen einer Verschlussklappe und gleichzeitige Betätigung eines Dosierstabes geschehen, der mit dem Abnehmen der Verschlussklappe in eine Entleerungsbereitschaftsstellung bringbar ist.

Die Person, die die Dosiervorrichtung nutzt, kann dann im weiteren Verlauf durch Anlegen eines Unterdrucks, das heißt durch orales Ansaugen den Luftstrom in der Dosiervorrichtung erzeugen, der die notwendigen Mechanismen innerhalb der Dosiervorrichtung bewirkt, um den Wirkstoff in einem Saugluftstrom fein zu verteilen und durch das Mundstück abzuleiten.

Eine derartige Dosiervorrichtung ist bereits aus der WO 2006/021546 A1 bekannt.

Insbesondere ist auch vorgeschlagen worden, einen Kolben vorzusehen, der durch Initiierung des Saugluftstroms zur Freigabe der Dosierkammer bewegt wird, so dass diese Dosierkammer, in der sich eine dosierte Menge des Wirkstoffs befindet, mit dem Saugluftstrom in Verbindung gebracht werden kann, um den Wirkstoff im Saugluftstrom zu verteilen.

Der vorliegenden Erfindung liegt vor dem Hintergrund des Standes der Technik die Aufgabe zugrunde, einen Mechanismus der Freigabe der Dosierkammer vorteilhaft auszugestalten und damit dazu beizutragen, dass die Dosierkammer in reproduzierbarer und äußerst zuverlässiger Weise zum richtigen Zeitpunkt während der Erzeugung des Saugluftstroms freigegeben wird.

Die Aufgabe wird vorteilhaft mit den Merkmalen des Patentanspruchs 1 gelöst.

Dabei weist die erfindungsgemäße Dosiervorrichtung einen ersten Gasströmungskanal sowie eine Dosierkammer auf, die sich zumindest zeitweise im Bereich des ersten Gasströmungskanals befindet sowie eine Dosierkammerfreigabeeinrichtung, welche ein zwischen einer Schließstellung und einer offenen Stellung verschiebbares Trennelement aufweist, so dass der erste Gasströmungskanal und/oder eine Verbindung zwischen dem ersten Gasströmungskanal und der Dosierkammer wahlweise versperrt oder freigegeben werden kann, wobei das Trennelement an einem Gleitführungselement zwischen der Schließstellung und der offenen Stellung verschiebbar und durch einen am Mundstück angelegten Unterdruck antreibbar ist und wobei das Trennelement und/oder das Gleitführungselement wenigstens im Gleitkontaktbereich mit einem die Gleitreibungseigenschaften beeinflussenden Gleitelement versehen ist.

Grundsätzlich sorgt das Gleitelement für definierte Gleitbedingungen zwischen dem Trennelement einerseits und dem Gleitführungselement andererseits. Dadurch wird ein Festhängen oder Verklemmen des antreibbaren Trennelementes verhindert und die Bedingungen für die Beschleunigung des Trennelementes mittels des Antriebes durch den Saugluftstrom werden reproduzierbar festgelegt, so dass auch der Zeitpunkt, an dem das Trennelement die Dosierkammer freigibt reproduzierbar eingehalten werden kann.

Dabei kann vorteilhaft vorgesehen sein, dass das Trennelement tulpenförmig ausgebildet ist und dass das Gleitelement in dem tulpenförmig erweiterten Teil des Trennelementes, insbesondere an dessen freiem Ende am umfangsseitigen Rand, vorgesehen ist.

Das Gleitelement kann vorteilhaft durch Anreicherung des Trennelementes und/oder des Gleitführungselementes wenigstens im Oberflächenbereich mit einem reibungsvermindernden oder reibungsbestimmenden Stoff gebildet sein.

Dabei können das Trennelement und/oder das Gleitführungselement diesen reibungsvermindernden/reibungsbestimmenden Stoff als Mischungsbestandteil enthalten oder mit diesem beschichtet sein.

Der Stoff kann als Pulver vorliegen und beispielsweise als Metallseife ausgebildet sein, insbesondere in Form von Magnesiumstearat.

Die Beschichtung mit einem derartigen Pulver hat den Vorteil, dass die reibungsbestimmende Wirkung schon bei Schichtdicken im µ-Bereich erreicht wird, so dass sich die Form der Reibungspartner durch die Beschichtung im wesentlichen nicht ändert und keine Nachbearbeitung notwendig ist. Besonders vorteilhaft sind solche Pulver einsetzbar, die eine spezifische Oberfläche zwischen 2 und 20 qm/ Gramm , insbesondere zwischen 2,3 und 9,3 qm/ Gramm , bevorzugt zwischen 3 und 8 qm/Gramm, weiter bevorzugt zwischen 3 und 5 qm/ Gramm aufweisen.

Eine weitere Ausführungsform der Erfindung sieht vor, dass das Gleitelement durch eine Vorsprünge aufweisende Oberflächenkontur gebildet ist. Dabei können die Vorsprünge als Noppen oder Stege, die insbesondere in Gleitrichtung verlaufen, ausgebildet sein. Die entsprechenden Vorsprünge sind vorteilhaft abgerundet und können auf dem Trennelement oder auch auf dem Gleitführungselement vorgesehen sein. Vorteilhaft können gleichmäßig über den Umfang verteilt 3 bis 20, vorzugsweise 6 bis 12 Noppen vorgesehen sein. Diese können am freien Ende des erweiterten Teils des tulpenförmigen Trennelementes angeordnet sein.

Es ist auch denkbar, gleichzeitig entsprechende Vorsprünge auszubilden und einen reibungsbestimmenden Zusatz als Mischungskomponente oder Beschichtung zusätzlich aufzubringen.

Im folgenden wird die Erfindung anhand eines Ausführungsbeispiels in einer Zeichnung gezeigt und nachfolgend beschrieben.

Dabei zeigt
- Figur 1: einen Vertikalschnitt durch eine Dosiervorrichtung, die mittels einer Abdeckkappe verschlossen ist;
- Figur 2: einen um 90° gedrehten Längsschnitt, ähnlich dem aus Figur 1;
- Figur 3: einen Längsschnitt der Dosiervorrichtung während des Abziehens der Abdeckkappe;
- Figur 4: den Zustand der Dosiervorrichtung nach Freigabe des ersten Gasströmungskanals durch die Dosiermittelfreigabeeinrichtung;
- Figur 5: eine dreidimensionale Außenansicht des Innenaufbaus der Dosiervorrichtung;
- Figur 6: eine dreidimensionale Innenansicht der Dosiervorrichtung aus einer von der Figur 5 verschiedenen Richtung;
- Figur 7: in dreidimensionaler Ansicht einen Dosierstab;
- Figur 8: in dreidimensionaler Darstellung ein Trennelement;
- Figur 9: ein Trennelement mit einem Gleitführungselement im Querschnitt,
- Figur 10: ein Trennelement in dreidimensionaler Ansicht in einer von der Figur 8 unterschiedlichen Ausgestaltung;
- Figur 11: ein Trennelement mit Vorsprüngen an seiner tulpenförmigen Erweiterung in einer Seitenansicht;
- Figur 12: das Trennelement aus Figur 11 in einer um 90 Grad gedrehten Seitenansicht;
- Figur 13: das Trennelement aus Figur 11 in einer geschnittenen Seitenansicht;
- Figur 14: das Trennelement in einer Draufsicht von der der Dosierkammer zugewandten Unterseite;
- Figur 15: das Trennelement aus Figur 11 in einer Draufsicht von der dem Mundstück zugewandten Oberseite und
- Figur 16: das Trennelement aus Figur 11 in einer 3- dimensionalen Ansicht.

Das in den Figuren dargestellte Dosiergerät zum Inhalieren eines Wirkstoffs 2 stellt ein für eine Person von Hand bedienbares zylindrisches, stabförmiges Taschengerät dar mit einem Gehäuse 3 und einer Verschlusskappe 7, die zu Beginn der Benutzung von dem Gerät abgenommen werden muss.

Dazu weist die Verschlusskappe 7 ein Innengewinde 8 auf, das mit einem Außengewinde 9 des Gehäuses 3 zusammenwirkt.

Beim Abschrauben der Verschlusskappe 7 wirken zudem Rippen 10 an einem Außenzylinder 4 des Gerätes mit Nuten 11 an der Innenseite der Verschlusskappe 7 zusammen, so dass beim Abdrehen die Verschlusskappe ein Teil der Dosiervorrichtung gegenüber dem Gehäuse 3 in Drehung versetzt und hierdurch ein Drehteil 28 gedreht wird, das mit einem als Pulverbrechvorrichtung wirkenden Rotor R, der asymmetrisch ausgebildet ist, in Verbindung steht, so dass bei jeder Benutzung der Wirkstoff 2 in der Vorratskammer 15 bewegt wird.

Im verschlossenen Zustand der Dosiervorrichtung taucht ein Dosierstab 33 in Form eines Dosierschwertes mit einer an seinem Endbereich befindlichen Dosierkammer 14, die als durchgehende, konische Öffnung ausgebildet ist, in die Vorratskammer 15 ein und wird im Zuge der Abschraubbewegung innerhalb der Vorratskammer gedreht. Hierdurch wird ein Auffüllen der Dosierkammer 14 mit dem Wirkstoff sichergestellt.

Der Dosierstab 33 weist an seinem der Dosierkammer 14 gegenüberliegenden Ende eine Andockstelle 41 mit einem Rastkopf 45 und einer Ringnut 46 auf, die in Verschlussstellung mit Nasen 47 eines mit der Verschlusskappe verbundenen, längsgeschlitzten Hohlzylinders 43 zusammenwirkt.

Dadurch kann mittels des während der Schraubbewegung durchlaufenden Axialhubs der Verschlusskappe 7 der Dosierstab, gezogen durch die Nasen 47 des Hohlzylinders 43 aus der Vorratskammer 15 vollständig herausgezogen werden, bis dass die Dosierkammer 14 im Bereich des Gasströmungskanals 60, 61, 62, 63, 68 liegt, der von einem Einlassbereich 60 über das Innere der Dosiervorrichtung bis zur Öffnung 48 des Mundstücks 6 führt.

Durch diesen ersten Gasströmungskanal wird nach Abnehmen der Verschlusskappe durch Ansaugen am Mundstück 6 Luft angesaugt und damit ein Teil-Gasstrom erzeugt.

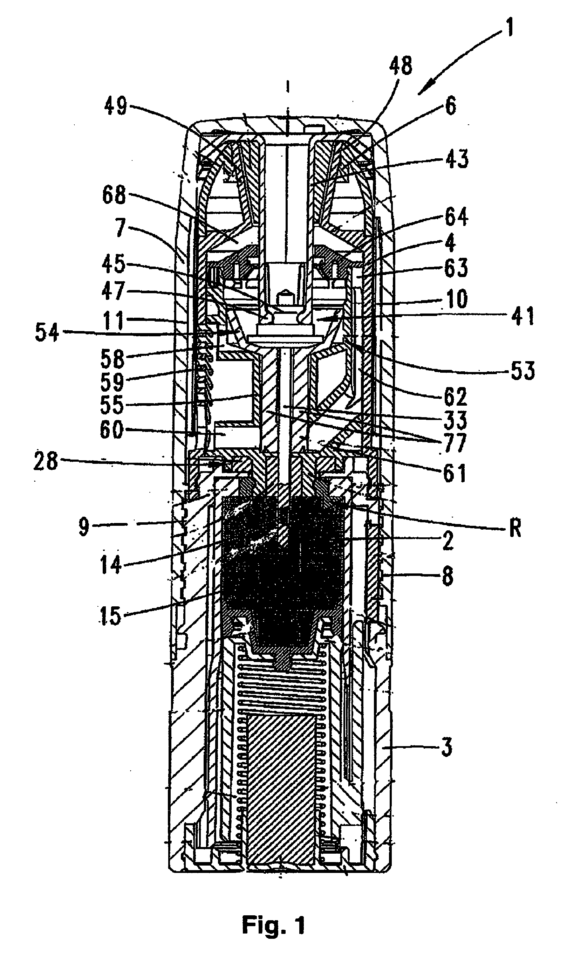

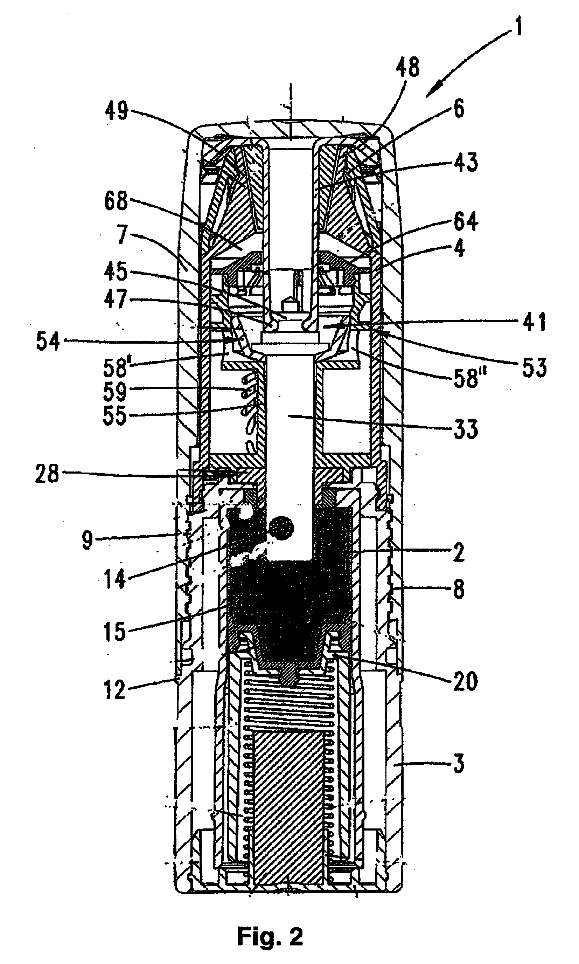

In der in Figur 1 dargestellten Position ist die Dosierkammer ebenso wie in der in Figur 2 gezeigten Position noch in der Vorratskammer 15 befindlich.

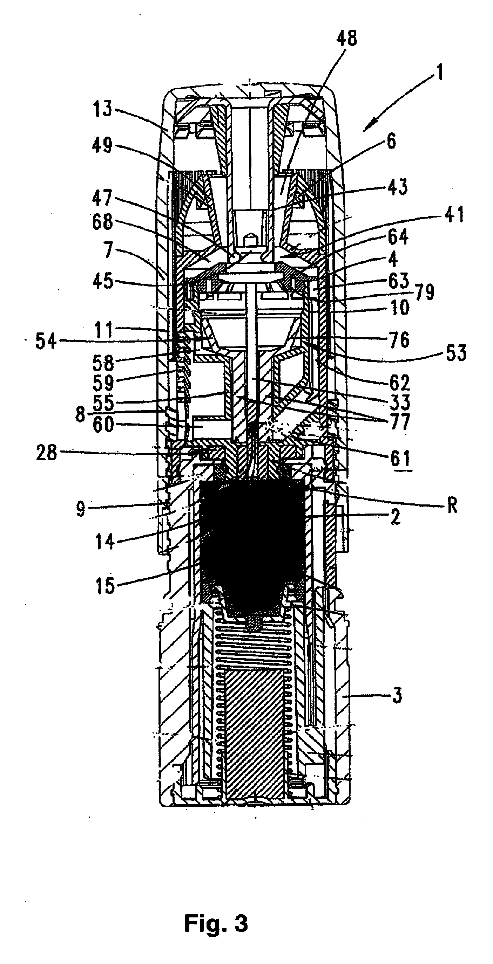

Figur 3 zeigt einen teilweise abgezogenen Zustand der Verschlusskappe 7, in dem der Dosierstab 33 bereits aus der Vorratskammer 15 vollständig herausbewegt ist.

In diesem Zustand ist allerdings die Dosierkammer 14 von dem ersten Gasströmungskanal 60, 61, 62, 63, 68 noch durch ein Trennelement 54, 76, 77 vollständig getrennt. Das Trennelement weist einen Kolben 54 auf, sowie einen Kolbenkopf 76 und beiderseits des Dosierstabes 33 verlaufende Zungen 77, die die Dosierkammer 14 in der dargestellten Position beidseitig abdecken.

Wird die Verschlusskappe 7 weiter abgedreht beziehungsweise abgezogen, so spreizen sich die Enden des Hohlzylinders 43 radial auf und die Nasen 47 gleiten aus der Ringnut 46, so dass die Verschlusskappe weiter vollständig abgenommen werden kann. Der Dosierstab 33 verbleibt dann in der ausgezogenen Position und wird dort durch die Rastfinger 79 des Deckelteils 64 festgehalten.

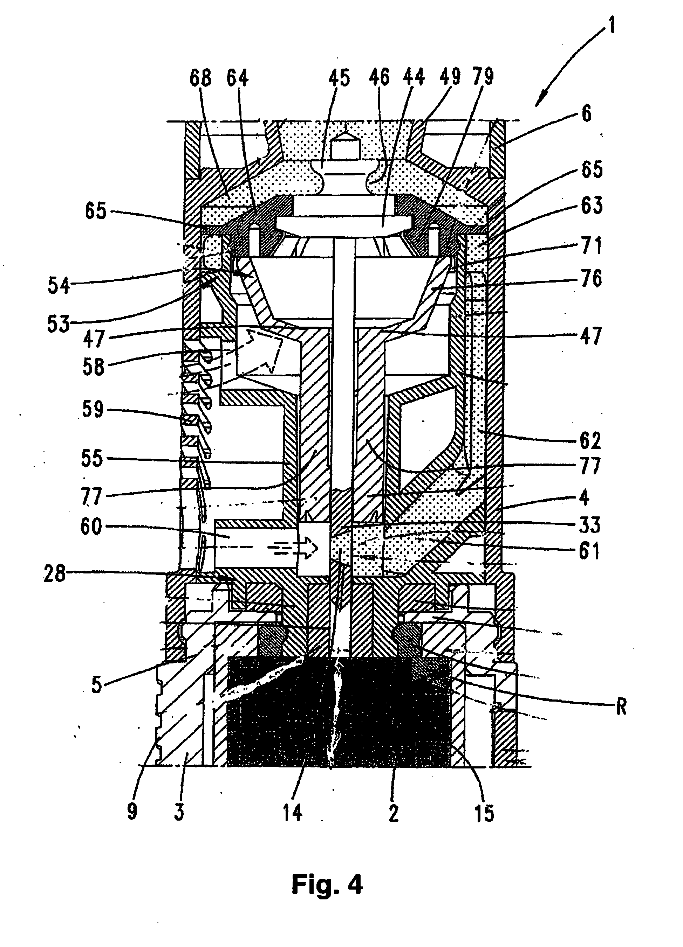

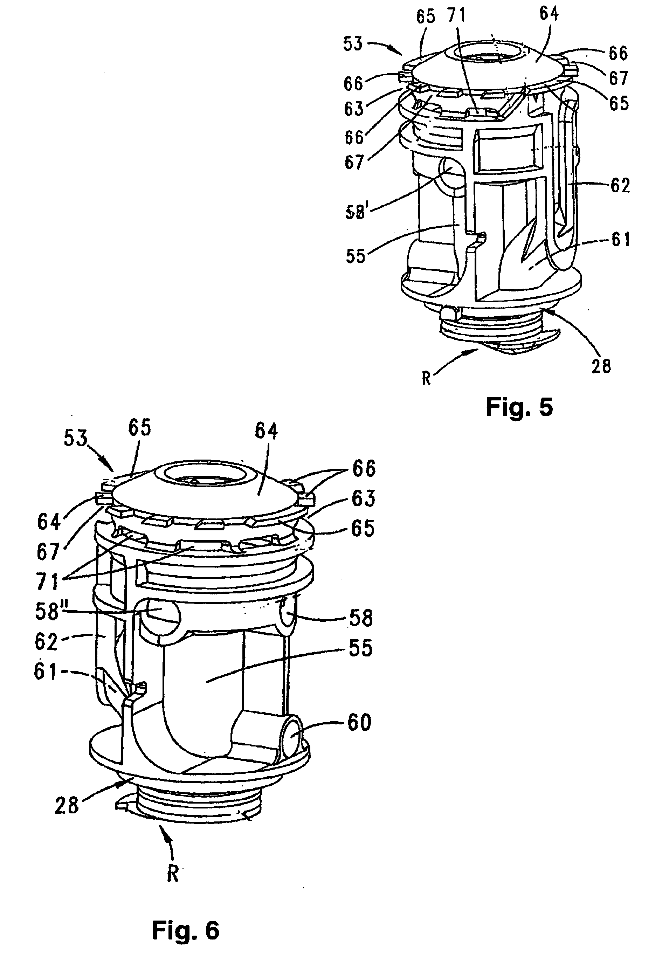

Wird nun nach Abziehen der Verschlusskappe an der Öffnung des Mundstücks 6 ein Saugluftstrom erzeugt, so seien dessen Folgen anhand der Figur 4 näher erläutert. Der Innenzylinder 53, der im übrigen in den Figuren 5 und 6 in verschiedenen Ansichten von außen dreidimensional dargestellt ist, ist im wesentlichen als Hohlkörper ausgebildet und führt zentral in einem hohlzylindrischen Führungsabschnitt 55 die Zungen 77 des Trennelements 54, 76, 77 in Form einer Gleitführung. An den Führungsabschnitt 55 schließt sich ein erweiterter Teil des Innenzylinders 53 an, in dem der Kolbenkopf 76 als tulpenförmige Erweiterung des Trennelementes sich bewegt. Nach Abziehen der Verschlusskappe ist der Dosierstab 33 durch Einziehen eines Radialkragens 44 hinter Rastfinger 79 des Deckelteils 64 festgehalten.

Wird über das Mundstück 6 Luft angesaugt, so wird zunächst durch die Öffnungen 71 in dem Innenzylinder 53 der Kolben 54 zum Mundstück hin angesaugt, bis er an der Unterseite des Deckelteils 64 anliegt. Von außen kann über Radialöffnungen 58, 58', 58" Luft nachströmen. Die Radialöffnungen stehen mit einem außenzylinderseitigen Gitterwandabschnitt 59 in Verbindung.

Der Auslösedruck des Trennelementes 54, 76, 77, bei dem dieses angezogen wird, kann mit Hilfe der erfindungsgemäßen Ausgestaltung der Gleitführung zuverlässig beispielsweise auf ein Kilopascal festgelegt werden. Dies gelingt durch Beschichtung , beispielsweise des Trennelementes, mit Magnesiumstearatpulvern, die eine spezifische Oberfläche zwischen 2 und 20 qm/ Gramm , insbesondere zwischen 2,3 und 9,3 qm/ Gramm , bevorzugt zwischen 3 und 8 qm/Gramm, weiter bevorzugt zwischen 3 und 5 qm/ Gramm aufweisen.

Die folgende Tabelle zeigt die bei der Erprobung der Erfindung ermittelten Versuchsergebnisse:

| **Kontaktfläche zwischen Trennelement und Gleitführungselement von in etwa 46,5 qmm ("Quadratmillimeter")** | **Benötigter Unterdruck (Druckdifferenz) zum Betätigen des Trennelements zur Freigabe der Dosierkammer** |
|---|---|
| keine Beschichtung (unbehandelt) | 3,5 bis 4,5 kPa ("Kilopascal") |
| Spezifische Oberfläche der MgSt-Beschichtung von in etwa 2,3 qm/gr | 0,5 bis 0,8 kPa |
| Spezifische Oberfläche der MgSt-Beschichtung von in etwa 9,3 qm/gr | 1,8 bis 2,1 kPa |

Eine ähnliche Wirkung kann durch das Vorsehen von Noppen oder Stegen erreicht werden.

Nach Anziehen des Trennelementes wird der den Kolbenkopf 76 umgebende Ringraum des Innenzylinders 53 durch einen aus den Radialöffnungen 58, 58' und 58" durchtretenden Hauptluftstrom durchströmt, der durch die Öffnungen 71 in die Ringkammer 63 eintritt.

Durch Bewegung des Kolbenkopfes 76 sind die Zungen 77 in Richtung zum Mundstück 6 hin verlagert und damit ist die Dosierkammer 14 freigegeben. Durch den ersten Gasströmungskanal 60 kann nun unter Passieren der Dosierkammer 14 ein Gasstrom in den Kanalzwischenabschnitt 61 und weiter axial über den Kanal 62 in die Ringkammer 63 strömen, der den fein verteilten Wirkstoff 2 aus der Dosierkammer 14 mitreißt und verteilt. Aus der Ringkammer 63 kann das dort mit dem weiteren Luftstrom aus den Öffnungen 58, 58', 58" vermischte Gas über Zwischenräume 67 an dem Deckelteil 64 zwischen Flügeln 65, 66 hindurch in den Ringraum 68 strömen, wobei eine Verwirbelung im Umfangsrichtung der Dosiervorrichtung entsteht, die eine weitere homogene Feinverteilung des Wirkstoffs im Gasstrom bewirkt. Der Gasstrom wird danach in einem sich zur Mundstücköffnung 48 hin erweiternden Dispergierteil 49 entspannt und gelangt darauf in den Mundraum der ansaugenden Person.

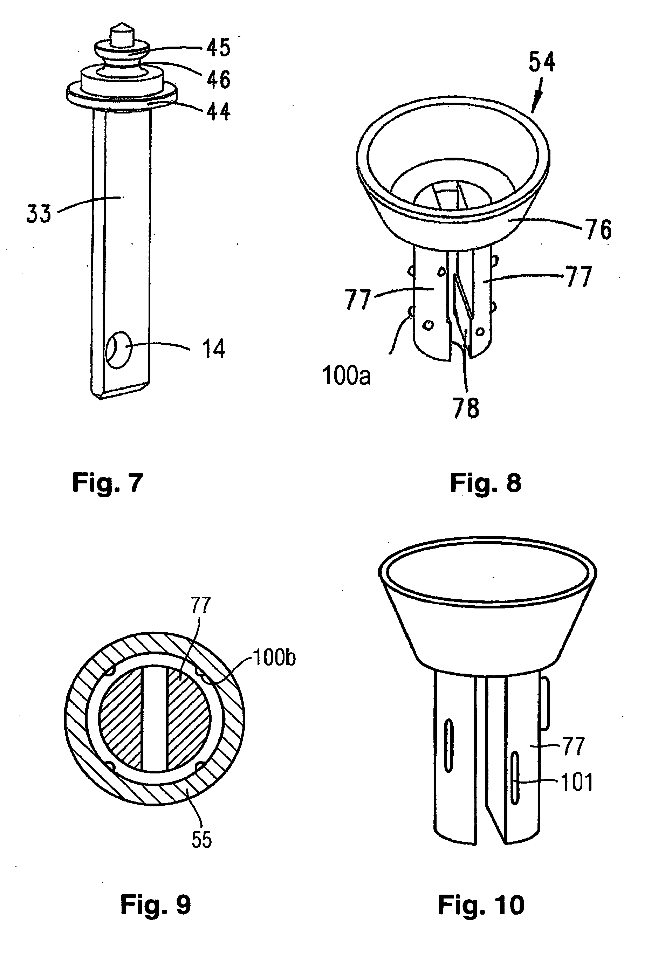

Die Figur 8 stellt weiter im Detail die Zungen 77 vor, die in ihrem Endbereich verstärkte Abdichtungsflächen 78 aufweisen, die in Schließstellung die Dosierkammer 14 zuverlässig abdichten, so dass auch bei Erschütterung der Dosiervorrichtung der Wirkstoff nicht aus dieser abrieseln kann.

Im Außenbereich am Umfang der Zungen 77 sind Noppen 100a in Form von kreisrunden, abgerundeten Erhebungen dargestellt. Die Erhebungen können jedoch, ebenso wie eine Anreicherung mit einem reibungsbeeinflussenden Stoff, auch im Bereich des Kolbens/der Erweiterung 54 des Trennelementes vorgesehen sein, wie weiter unten erläutert. Die Erhebungen dienen ebenso wie eine alternativ oder zusätzlich aufgebrachte Magnesiumstearatschicht der Reduzierung der Reibung und/oder der genaueren Reproduzierbarkeit der Reibungsbedingungen. Ebenso kann Magnesiumstearat dem aus einem Kunststoff bestehenden Trennelement als Mischungsbestandteil beigefügt werden. Es werden typisch Pulver mit einer spezifischen Oberfläche zwischen 2,3 und 9,3 m2/g verwendet und bei einer Beschichtung ergeben sich entsprechend Schichtdicken im Mikrometerbereich.

Figur 9 stellt im Querschnitt ein Trennelement mit den Zungen 77 dar, die in dem Führungsabschnitt 55 liegen, wobei dort der Führungsabschnitt 55 mit kreisrundem Querschnitt Noppen 100b zur genaueren Definierung der Gleiteigenschaften aufweist.

Die Figur 10 schließlich zeigt als eine Ausführungsform der Erfindung längliche, in Bewegungsrichtung des Trennelementes verlaufende Stege 101, die am Umfang der Zungen 77 verteilt sind und an der Innenfläche des Führungsabschnitts 55 gleiten.

Die oben dargestellte und im einzelnen beschriebene Verwendung reibungsdefinierender Mittel in Form einer Beschichtung des Führungsabschnitts 55 oder der Zungen 77 oder beider Teile mittels Metallseifen, insbesondere Magnesiumstearat oder das Vorsehen von Noppen im Umfangsbereich des Trennelementes insbesondere am Umfang der Zungen 77 bewirken eine zuverlässige und reproduzierbare Festlegung der Reibungseigenschaften beim Gleiten des Trennelementes in dem Führungsabschnitt und garantieren damit ein zuverlässiges Auslösen des Trennelementes und eine zeitlich genau festgelegte Freigabe der Dosierkammer, so dass die Funktion der Dosiereinrichtung bei der Erzeugung eines mit einem Wirkstoff angereicherten Gasstroms garantiert ist.

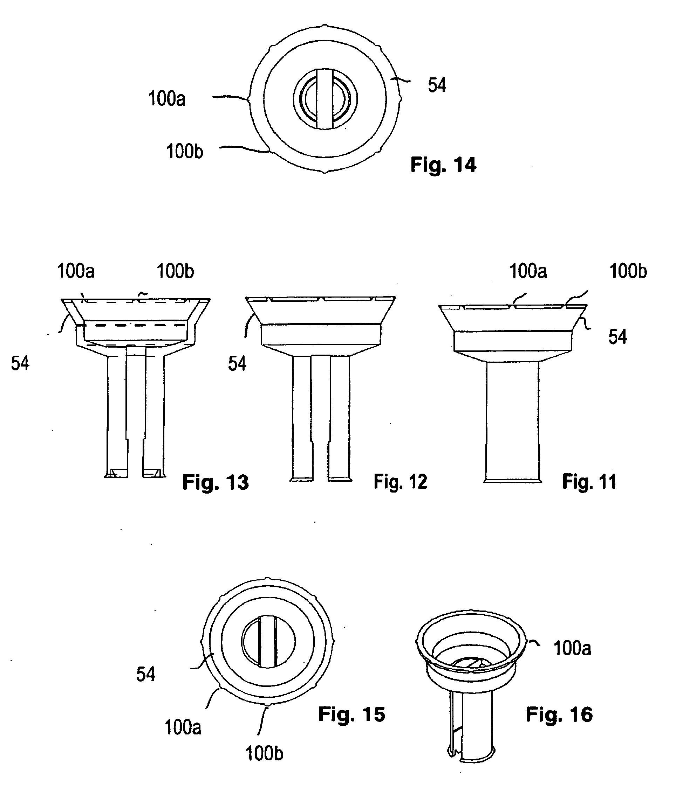

Die Figur 11 zeigt eine Ausführungsform des Trennelementes 54,76,77 , bei dem die tulpenförmige Erweiterung in Form des Kolbens 54 radial herausragende Noppen 100a, 100b aufweist, die an dem Innenzylinder 53 in dessen erweitertem Bereich gleitend geführt sind. Vorteilhaft können 3 bis 20 Noppen, insbesondere 6 bis 12 Noppen vorgesehen sein. Die Noppen können am oberen, äußeren Rand der Erweiterung des Trennelementes, dort, wo diese an dem Gleitführungselement in Form des Innenzylinders 53 direkt anliegt, vorgesehen sein.

Durch die Verteilung der Noppen und somit der Gleitführung auf einem kurzen Höhenabschnitt des Trennelementes wird ein Verkanten des Trennelementes 54,76,77 im Gleitführungselement 53,55 effektiv verhindert.

Auch eine Beschichtung mit einem reibungssteuemden- oder vermindernden Stoff, wie z.B. Magnesiumstearat kann auf diesen Bereich beschränkt sein oder sich zumindest in diesen Bereich erstrecken.

Der mit den Noppen bzw. der Beschichtung versehene Bereich beträgt dabei in etwa 40 bis 50 qmm. Dies hat sich im Experiment, vgl. vorstehende Tabelle, als besonders günstig hinsichtlich des benötigten Auslöseunterdrucks herausgestellt.

Die Figuren 12 bis 16 zeigen verschiedene Ansichten derselben Ausführungsform wie Figur 11 aus unterschiedlichen Perspektiven, wobei eine vorzugsweise symmetrische Verteilung der Noppen/Erhöhungen am Umfang des Trennelementes und eine Anordnung in einer einzigen Reihe auf gleicher Höhe deutlich wird.

### Bezugszeichenliste

- 1: Dosiervorrichtung
- 2: Wirkstoff
- 3: Gehäuse
- 4: Außenzylinder
- 6: Mundstück
- 7: Verschlussklappe
- 8: Innengewinde
- 9: Außengewinde
- 10: Rippen
- 11: Nuten
- 14: Dosierkammer
- 15: Vorratskammer
- 28: Drehteil
- 33: Dosierstab
- 41: Andockstelle
- 43: Hohlzylinder
- 44: Radialkragen
- 45: Rastkopf
- 46: Ringnut
- 47: Nasen
- 48: Mundstücköffnung
- 49: Dispergierteil
- 53: Innenzylinder
- 54: Kolben
- 55: Führungsabschnitt
- 53, 55: Gleitführungselement
- 58, 58', 58": Radialöffnungen
- 59: Gitterwandabschnitt
- 60: Strömungskanal
- 61: Kanalzwischenabschnitt
- 62: Kanal
- 63: Ringkammer
- 64: Deckelteil
- 65, 66: Flügel
- 67: Zwischenräume
- 68: Ringraum
- 71: Öffnungen
- 76: Kolbenkopf
- 77: Zungen
- 79: Rastfinger
- 100a, 100b: Noppen
- 101: Stege

- R: Rotor

## Patentansprüche

1. Dosiervorrichtung (1) zur Erzeugung eines Gasstromes mit einem, in diesem fein verteilten Wirkstoff (2) in Folge eines an einem Mundstück (6) der Dosiervorrichtung angelegten Unterdrucks mit einem ersten Gasströmungskanal (60, 61, 62, 63, 68) und mit einer Dosierkammer (14), die sich zumindest zeitweise im Bereich des ersten Gasströmungskanals (60, 61, 62, 63, 68) befindet sowie mit einer Dosierkammerfreigabeeinrichtung, welche ein zwischen einer Schließstellung und einer offenen Stellung verschiebbares Trennelement (54, 76, 77) aufweist, wobei in der Schließstellung des Trennelementes (54, 76, 77) der erste Gasströmungskanal (60, 61, 62, 63, 68) und/oder eine Verbindung zwischen dem ersten Gasströmungskanal und der Dosierkammer (14) versperrt und in der offenen Stellung des Trennelementes(54, 76, 77) der erste Gasströmungskanal (60, 61, 62, 63, 68) und/ oder die Verbindung zwischen dem ersten Gasströmungskanal und der Dosierkammer freigegeben ist, und mit einem Gleitführungselement (53, 55), an dem das Trennelement (54, 76, 77) zwischen der Schließstellung und der offenen Stellung verschiebbar und durch den am Mundstück (6) angelegten Unterdruck antreibbar ist, und wobei das Trennelement (54, 76, 77) und/oder das Gleitführungselement (53, 55) wenigstens im Gleitkontaktbereich mit einem die Gleitreibungseigenschaften beeinflussenden Gleitelement (100a, 100b, 101) versehen ist.

2. Dosiervorrichtung nach Anspruch 1,
**dadurch gekennzeichnet, dass** das Trennelement (54, 76, 77) tulpenförmig ausgebildet ist und dass das Gleitelement (100a, 100b, 101) in dem tulpenförmig erweiterten Teil (76) des Trennelementes (54, 76, 77), insbesondere an dessen freiem Ende, vorgesehen ist.

3. Dosiervorrichtung nach Anspruch 1 oder 2,
**dadurch gekennzeichnet, dass** das Gleitelement (100a, 100b, 101) durch eine Anreicherung des Trennelementes (54, 76, 77) und/oder des Gleitführungselementes (53, 55) im Oberflächenbereich mit einem reibungsvermindernden Stoff gebildet ist.

4. Dosiervorrichtung nach Anspruch 3,
**dadurch gekennzeichnet, dass** das Trennelement (54, 76, 77) und/oder das Gleitführungselement (53, 55) den reibungsvermindemden Stoff als Mischungsbestandteil enthalten.

5. Dosiervorrichtung nach Anspruch 3,
**dadurch gekennzeichnet, dass** das Trennelement (54, 76, 77) und/oder das Gleitführungselement (53, 55) mit einem reibungsvermindemden Stoff beschichtet sind.

6. Dosiervorrichtung nach Anspruch 3, 4 oder 5,
**dadurch gekennzeichnet, dass** der reibungsvermindernde Stoff ein Pulver ist.

7. Dosiervorrichtung nach Anspruch 2, 3, 4 oder 5,
**dadurch gekennzeichnet, dass** der reibungsvermindernde Stoff eine Metallseife, insbesondere Magnesiumstearat ist.

8. Dosiervorrichtung nach Anspruch 7,
**dadurch gekennzeichnet, dass** die Metallseife, insbesondere das Magnesiumstearatpulver, eine spezifische Oberfläche zwischen 2 und 20 qm/ g , insbesondere zwischen 3 und 8 qm/g, weiter bevorzugt zwischen 3 und 5 qm/ g aufweist.

9. Dosiervorrichtung nach Anspruch 1oder 2,
**dadurch gekennzeichnet, dass** das Gleitelement (100a, 100b, 101) durch eine Vorsprünge aufweisende Oberflächenkontur gebildet ist.

10. Dosiervorrichtung nach Anspruch 9"
**dadurch gekennzeichnet, dass** die Oberflächenkontur Noppen (100a,100b) aufweist, wobei die Zahl der Noppen insbesondere zwischen 2 und 21 liegt.

11. Dosiervorrichtung nach Anspruch 10,
**dadurch gekennzeichnet, dass** die Zahl der Noppen(100a,100b) zwischen 5 und 13 liegt.

12. Dosiervorrichtung nach Anspruch 9,
**dadurch gekennzeichnet, dass** die Oberflächenkontur im wesentlichen in Gleitrichtung verlaufende Stege (101) aufweist.

13. Dosiervorrichtung nach Anspruch 9, 10,11 oder 12,
**dadurch gekennzeichnet, dass** die Vorsprünge im Bereich des Kontaktes mit dem Gleitpartner abgerundet sind.

14. Dosiervorrichtung nach einem der Ansprüche 9 bis 13,
**dadurch gekennzeichnet, dass** die Vorsprünge jeweils gleiche Höhe aufweisen.

15. Dosiervorrichtung nach Anspruch 1 oder 2,
**dadurch gekennzeichnet, dass** das Gleitelement (100a, 100b, 101) durch eine Vorsprünge aufweisende Oberflächenkontur sowie zusätzlich durch eine Anreicherung des Trennelementes (54, 76, 77) und/oder des Gleitführungselementes (53, 55) im Oberflächenbereich mit einem reibungsvermindernden Stoff gebildet ist.

## Claims

1. Dosing device (1) for generating a gas flow with an active substance (2) finely dispersed in said gas flow as a result of a negative pressure applied to a mouthpiece (6) of the dosing device,
said dosing device having a first gas flow channel (60, 61, 62, 63, 68) and a dosing chamber (14), which is situated at least sometimes in the region of the first gas flow channel (60, 61, 62, 63, 68), as well as a dosing chamber release mechanism which has a separating element (54, 76, 77) which can be displaced between a closed position and an open position,
wherein, in the closed position of the separating element (54, 76, 77), the first gas flow channel (60, 61, 62, 63, 68) and/or a connection between the first gas flow channel and the dosing chamber (14) is blocked and, in the open position of the separating element (54, 76, 77), the first gas flow channel (60, 61, 62, 63, 68) and/or the connection between the first gas flow channel and the dosing chamber is released, and having a slide guide element (53, 55) on which the separating element (54, 76, 77) is displaceable between the closed position and the open position and on which the separating element (54, 76, 77) is driveable by means of the negative pressure applied at the mouthpiece (6), and wherein the separating element (54, 76, 77) and/or the slide guide element (53, 55) is provided at least in the sliding contact region with a slide element (100a, 100b, 101) that influences the sliding friction properties.

2. Dosing device according to Claim 1, **characterized in that** the separating element (54, 76, 77) is tulip-shaped and **in that** the slide element (100a, 100b, 101) is provided **in that** part (76) of the separating element (54, 76, 77) that is widened in a tulip-shaped manner, in particular at the free end thereof.

3. Dosing device according to Claim 1 or 2, **characterized in that** the slide element (100a, 100b, 101) is formed by an enhancement of the separating element (54, 76, 77) and/or of the slide guide element (53, 55) in the surface region with a friction-reducing substance.

4. Dosing device according to Claim 3, **characterized in that** the separating element (54, 76, 77) and/or the slide guide element (53, 55) contain the friction-reducing substance as an ingredient of the mixture.

5. Dosing device according to Claim 3, **characterized in that** the separating element (54, 76, 77) and/or the slide guide element (53, 55) are coated with a friction-reducing substance.

6. Dosing device according to Claim 3, 4 or 5, **characterized in that** the friction-reducing substance is a powder.

7. Dosing device according to Claim 2, 3, 4 or 5, **characterized in that** the friction-reducing substance is a metal soap, in particular magnesium stearate.

8. Dosing device according to Claim 7, **characterized in that** the metal soap, in particular the magnesium stearate powder, has a specific surface between 2 and 20 square m/g, in particular between 3 and 8 square m/g, further preferred between 3 and 5 square m/g.

9. Dosing device according to Claim 1 or 2, **characterized in that** the slide element (100a, 100b, 101) is formed by a surface contour that has projections.

10. Dosing device according to Claim 9, **characterized in that** the surface contour has knobs (100a, 100b), wherein the number of knobs is in particular between 2 and 21.

11. Dosing device according to Claim 10, **characterized in that** the number of knobs (100a, 100b) is between 5 and 13.

12. Dosing device according to Claim 9, **characterized in that** the surface contour has webs (101) which extend substantially in the sliding direction.

13. Dosing device according to Claim 9, 10, 11 or 12, **characterized in that** the projections are rounded in the region of contact with the sliding partner.

14. Dosing device according to one of Claims 9 to 13, **characterized in that** the height of the projections in each case is identical.

15. Dosing device according to Claim 1 or 2, **characterized in that** the slide element (100a, 100b, 101) is formed by a surface contour having projections and additionally by an enhancement of the separating element (54, 76, 77) and/or of the slide guide element (53, 55) in the surface region with a friction-reducing substance.

## Revendications

1. Dispositif de dosage (1) destiné à générer un flux gazeux, avec un principe actif (2) finement distribué dans celui-ci, du fait d'une dépression appliquée sur un embout buccal (6) du dispositif de dosage, avec un premier canal d'écoulement de gaz (60, 61, 62, 63, 68) et avec une chambre de dosage (14) qui se trouve au moins temporairement dans la région du premier canal d'écoulement de gaz (60, 61, 62, 63, 68), ainsi qu'avec un système de libération de la chambre de dosage, lequel comporte un élément séparateur (54, 76, 77) déplaçable entre une position de fermeture et une position ouverte, dans la position de fermeture de l'élément séparateur (54, 76, 77), le premier canal d'écoulement de gaz (60, 61, 62, 63, 68) et/ou une liaison entre le premier canal d'écoulement de gaz et la chambre de dosage étant fermé(e) et dans la position ouverte de l'élément de séparation (54, 76, 77), le premier canal d'écoulement de gaz (60, 61, 62, 63, 68) et/ou la liaison entre le premier canal d'écoulement de gaz et la chambre de dosage étant libéré(e), avec un élément de guidage (53, 55) lisse sur lequel l'élément séparateur (54, 76, 77) est déplaçable entre la position de fermeture et la position ouverte et par lequel la dépression appliquée sur l'embout buccal (6) peut être amorcée et l'élément séparateur (54, 76, 77) et/ou l'élément de guidage (53, 55) lisse étant muni au moins dans la région de contact glissant d'un élément de glissement (100a, 100b, 101) influençant le comportement au glissement.

2. Dispositif de dosage selon la revendication 1, **caractérisé en ce que** l'élément séparateur (54, 76, 77) est conçu en forme de tulipe et **en ce que** l'élément de glissement (100a, 100b, 101) est prévu dans la partie (76) évasée en forme de tulipe de l'élément séparateur (54, 76, 77), notamment sur l'extrémité libre de celui-ci.

3. Dispositif de dosage selon la revendication 1 ou la revendication 2, **caractérisé en ce que** l'élément de glissement (100a, 100b, 101) est formé par un enrichissement de l'élément séparateur (54, 76, 77) et/ou de l'élément de guidage (53, 55) lisse dans la zone superficielle, d'une substance réductrice de frottement.

4. Dispositif de dosage selon la revendication 3, **caractérisé en ce que** l'élément séparateur (54, 76, 77) et/ou l'élément de guidage (53, 55) lisse contiennent la substance réductrice de frottement en tant que composant de mélange.

5. Dispositif de dosage selon la revendication 3, **caractérisé en ce que** l'élément séparateur (54, 76, 77) et/ou l'élément de guidage (53, 55) lisse sont revêtus d'une substance réductrice de frottement.

6. Dispositif de dosage selon la revendication 3, la revendication 4 ou la revendication 5, **caractérisé en ce que** la substance réductrice de frottement est une poudre.

7. Dispositif de dosage selon la revendication 2, la revendication 3, la revendication 4 ou la revendication 5, **caractérisé en ce que** la substance réductrice de frottement est un savon métallique, notamment du stéarate de magnésium.

8. Dispositif de dosage selon la revendication 7, **caractérisé en ce que** le savon métallique, notamment la poudre de stéarate de magnésium présente une surface spécifique comprise entre 20 et 20 qm/g, notamment entre 3 et 8 qm/g, de manière plus préférée entre 3 et 5 qm/g.

9. Dispositif de dosage selon la revendication 1 ou la revendication 2, **caractérisé en ce que** l'élément de glissement (100a, 100b, 101) est formé par un contour superficiel comportant des saillies.

10. Dispositif de dosage selon la revendication 9, **caractérisé en ce que** le contour superficiel comporte des noppes (100a, 100b), le nombre de noppes se situant notamment entre 2 et 21.

11. Dispositif de dosage selon la revendication 10, **caractérisé en ce que** le nombre de noppes (100a, 100b) se situe entre 5 et 13.

12. Dispositif de dosage selon la revendication 9, **caractérisé en ce que** le contour superficiel comporte des barrettes (101) s'étendant sensiblement dans la direction de glissement.

13. Dispositif de dosage selon la revendication 9, la revendication 10, la revendication 11, ou la revendication 12, **caractérisé en ce que** dans la région du contact avec la surface associée, les saillies sont arrondies.

14. Dispositif de dosage selon l'une quelconque des revendications 9 à 13, **caractérisé en ce que** les saillies présentent chaque fois la même hauteur.

15. Dispositif de dosage selon la revendication 1 ou la revendication 2, **caractérisé en ce que** l'élément de glissement (100a, 100b, 101) est formé par un contour superficiel comportant des saillies, ainsi qu'en supplément par un enrichissement de l'élément séparateur (54, 76, 77) et/ou de l'élément de guidage (53, 55) lisse dans la région superficielle d'une substance réductrice de frottement.
